# EUROPEAN PATENT SPECIFICATION

(11) **EP 3 482 641 B1**
(45) Date of publication and mention of the grant of the patent: **01.01.2025**
(21) Application number: 18200200.6
(22) Date of filing: 02.04.2015
(51) Int. Cl.: A24B 15/28, A24B 3/18, A24B 15/42

(54) **TOBACCO RAW MATERIAL**
TABAKROHMATERIAL
MATÉRIAU BRUT À BASE DE TABAC

(30) Priority: 04.04.2014 SE 1450421
(43) Date of publication of application: 15.05.2019
(62) Divisional of application: 15716994.7
(73) Proprietor: British American Tobacco Sweden AB, 215 32 Malmö (SE)
(72) Inventor: BJÖRKHOLM, Lars, SE-520 11 Vegby (SE)
(74) Representative: Gill, Siân Victoria

(56) References cited:
- US-A1- 2012 199 145
- US-A1- 2013 276 801
- TALHOUT R ET AL: "Sugars as tobacco ingredient: Effects on mainstream smoke composition", FOOD AND CHEMICAL TOXICOLOGY, PERGAMON, GB, vol. 44, no. 11, 1 November 2006 (2006-11-01), pages 1789 - 1798, XP027918547, ISSN: 0278-6915, [retrieved on 20061101]
- J C LEFFINGWE: "leaf Chemistry BA Basic Chemical Constituents of Tobacco Leaf and Differences among Tobacco Types", BLACKWELL SCIENCE (PUB.), 1 January 1999 (1999-01-01), XP055326787, Retrieved from the Internet <URL:http://www.leffingwell.com/download/Leffingwell> [retrieved on 20171003]

## Description

The present invention relates to a tobacco raw material, a smoking tobacco composition comprising the tobacco raw material, and a smokeless tobacco composition comprising the tobacco raw material.

The invention also relates to nasal snuff and oral smokeless tobacco products comprising the smokeless tobacco composition.

A process for production of a tobacco raw material, and to tobacco raw material obtainable by this process are also disclosed.

### Background

Tobacco fermentation is basically a process that starts spontaneously soon after the tobacco is harvested.

As tobacco fermentation was early on found to enhance certain properties of the tobacco, such as smell and taste, this spontaneous process is frequently consciously stimulated in various industrial processes in the course of tobacco production.

However, tobacco fermentation - spontaneous as well as industrially stimulated - also has the effect of generating undesired byproducts, such as for instance tobacco-specific nitrosamines, abbreviated TSNA, which are known to be carcinogens.

EP0075128 discloses a desmutagen, comprising, as an essential ingredient, sulfurous anhydride (SO₂), or an alkali metal, alkaline-earth metal, aluminium or ammonium salt of sulfite, hydrogen sulfite, pyrosulfite or dithionite, for inactivating mutagens contained in foods, foodstuffs, food additives, beverages and tobaccos.

Apart from examples showing treatment of tobacco tar, which of course is not a tobacco raw material but typically resinous, partially combusted particulate matter produced by the burning of tobacco in the act of smoking, it is only mentioned that a water solution of the above salts or a flavoring sherry into which the salts have been dissolved is applied to tobacco leaves. Neither tobacco fermentation, nor its relation to fermentable carbohydrates is mentioned in this document.

US3845774 discloses a method for curing tobacco by homogenizing harvested leaves. It is said that by the method undesirable components can be reduced in amount or completely eliminated by physical, chemical or biological means or combinations of such means. It is, however, also said that the cured material can be easily stored for aging or other processing such as fermentation prior to being reconstituted for manufacture into a smoking product. Hence, this document does not recognize fermentation as something problematic.

US20130276801 discloses chemical pulping of tobacco and bleaching the tobacco pulp to produce a dissolving grade pulp. Bleaching the tobacco pulp may include chlorination of the tobacco pulp with a chlorine dioxide solution, and caustic extraction of the tobacco pulp with a second strong base. Carbohydrate content is not suggested to be reduced or limited, but on the contrary to be protected during the treatments. Fermentation is not discussed in the document.

### Short summary of the invention

It would be desirable to be able to provide a tobacco raw material that can easily be provided with such desirable properties as is associated with tobacco fermentation, such as enhanced smell and taste, while not being burdened by the undesired side effects of tobacco fermentation.

One object of the present invention is to provide such a tobacco raw material.

Thus, one aspect of the invention relates to a bleached tobacco raw material
comprising less than 4 weight-% fermentable carbohydrates, calculated on the dry total weight of the bleached tobacco raw material, wherein the bleached tobacco raw material comprises less than 5.0 µg/kg of B(a)P (Benzo(a)pyrene); and one or more, but not all of: less than 7.0 mg/kg of nitrite; less than 2.0 mg/kg of a combined amount of NNN (N-nitrosonornicotine) and NNK ((4- methylnitrosamino)-1-(3-pyridyl)-1-butanone); less than 10.0 µg/kg of NDMA (N-Nitrosodimethylamine); less than 1.0 mg/kg of Cd; less than 2.0 mg/kg of Pb; less than 0.5 mg/kg of As; less than 3.0 mg/kg of Ni; and less than 4.5 mg/kg of Cr; calculated on the dry total weight of the bleached tobacco raw material.

### Description of embodiments of the invention

Before the present invention is disclosed and described, it is to be understood that this invention is not limited to the particular configurations, process steps, and materials disclosed herein as such configurations, process steps, and materials may vary somewhat. It is also to be understood that the terminology employed herein is used for the purpose of describing particular embodiments only and is not intended to be limiting since the scope of the present invention will be limited only by the appended claims.

It must be noted that, as used in this specification and the claims, the singular forms "a", "an", and "the" include plural referents unless the context clearly dictates otherwise.

In this specification, unless otherwise stated, the term "about" modifying the quantity of an ingredient in the tobacco raw material, tobacco compositions, or tobacco products of the invention or employed in the methods of the invention refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the tobacco raw material, tobacco compositions, or tobacco products, or to carry out the methods; and the like. The term "about" also encompasses amounts that differ due to different equilibrium conditions for a composition resulting from a particular initial mixture. Whether or not modified by the term "about", the claims include equivalents to the quantities. In this specification, unless otherwise stated, the term "bleached" refers to an object, such as for instance a tobacco raw material, whose visible color has been reduced by any known method for this purpose, such as washing with one or more solvents, or treatment with one or more bleaching agents, or any combination thereof.

In this specification, unless otherwise stated, the term "fermentable carbohydrates" refers to carbohydrates that can be metabolically broken down in and by the human body, specifically starch, glucose, fructose and sucrose.

In this specification, unless otherwise stated, the term "oral" in connection to a product refers to the product, in normal use, is suited to be placed somewhere in the oral cavity of the user, for example under the lips, in the same way as moist snuff products are generally used.

In one embodiment the bleached tobacco raw material comprises less than about 2 weight-%, specifically less than about 1 weight-%, of fermentable carbohydrates, calculated on the dry total weight of the bleached tobacco raw material.

In one embodiment the bleached tobacco raw material comprises less than about 7.0 mg/kg, specifically less than about 3.5 mg/kg, more specifically less than about 1.0 mg/kg of nitrite, calculated on the dry total weight of the bleached tobacco raw material.

In one embodiment the bleached tobacco raw material comprises a combined amount of NNN (N-nitrosonornicotine) and NNK ((4-methylnitrosamino)-1-(3-pyridyl)-1-butanone) that is less than about 2.0 mg/kg, specifically less than about 1.0 mg/kg, more specifically less than about 0.2 mg/kg, calculated on the dry total weight of the bleached tobacco raw material.

In one embodiment the bleached tobacco raw material comprises less than about 10.0 µg/kg, specifically less than about 5.0 µg/kg, more specifically less than about 0.6 µg/kg of NDMA (N-Nitrosodimethylamine), calculated on the dry total weight of the bleached tobacco raw material.

In one embodiment the bleached tobacco raw material comprises less than about 5.0 µg/kg, specifically less than about 2.5 µg/kg, more specifically less than about 0.6 µg/kg of B(a)P (Benzo(a)pyrene), calculated on the dry total weight of the bleached tobacco raw material.

In one embodiment the bleached tobacco raw material comprises less than about 1.0 mg/kg, specifically less than about 0.5 mg/kg, more specifically less than about 0.1 mg/kg of Cd, calculated on the dry total weight of the bleached tobacco raw material.

In one embodiment the bleached tobacco raw material comprises less than about 2.0 mg/kg, specifically less than about 1.0 mg/kg, more specifically less than about 0.1 mg/kg of Pb, calculated on the dry total weight of the bleached tobacco raw material.

In one embodiment the bleached tobacco raw material comprises less than about 0.5 mg/kg, specifically less than about 0.25 mg/kg, more specifically less than about 0.1 mg/kg of As, calculated on the dry total weight of the bleached tobacco raw material.

In one embodiment the bleached tobacco raw material comprises less than about 3.0 mg/kg, specifically less than about 1.5 mg/kg, more specifically less than about 0.1 mg/kg of Ni, calculated on the dry total weight of the bleached tobacco raw material.

In one embodiment the bleached tobacco raw material comprises less than about 4.5 mg/kg, specifically less than about 2.25 mg/kg, more specifically less than about 0.1 mg/kg of Cr, calculated on the dry total weight of the bleached tobacco raw material.

In one embodiment the bleached tobacco raw material comprises one or more but not all of:
less than about 7.0 mg/kg nitrite;
less than about 2.0 mg/kg combined amount of NNN (N-nitrosonornicotine) and NNK ((4-methylnitrosamino)-1-(3-pyridyl)-1-butanone);
less than about 10.0 µg/kg NDMA (N-Nitrosodimethylamine);
less than about 5.0 µg/kg B(a)P (Benzo(a)pyrene);
less than about 1.0 mg/kg Cd;
less than about 2.0 mg/kg Pb;
less than about 0.5 mg/kg As;
less than about 3.0 mg/kg Ni;
less than about 4.5 mg/kg Cr;
calculated on the dry total weight of the bleached tobacco raw material.

In one embodiment the bleached tobacco raw material comprises one or more but not all of:
less than about 3.5 mg/kg nitrite;
less than about 1.0 mg/kg combined amount of NNN (N-nitrosonornicotine) and NNK ((4-methylnitrosamino)-1-(3-pyridyl)-1-butanone);
less than about 5.0 µg/kg NDMA (N-Nitrosodimethylamine);
less than about 2.5 µg/kg B(a)P (Benzo(a)pyrene);
less than about 0.5 mg/kg Cd;
less than about 1.0 mg/kg Pb;
less than about 0.25 mg/kg As;
less than about 1.5 mg/kg Ni;
less than about 2.25 mg/kg Cr;
calculated on the dry total weight of the bleached tobacco raw material.

In one embodiment the bleached tobacco raw material comprises one or more but not all of:
less than about 1.0 mg/kg nitrite;
less than about 0.2 mg/kg combined amount of NNN (N-nitrosonornicotine) and NNK ((4-methylnitrosamino)-1-(3-pyridyl)-1-butanone);
less than about 0.6 µg/kg NDMA (N-nitrosodimethylamine);
less than about 0.6 µg/kg B(a)P (benzo(a)pyrene);
less than about 0.1 mg/kg Cd;
less than about 0.1 mg/kg Pb;
less than about 0.1 mg/kg As;
less than about 0.1 mg/kg Ni;
less than about 0.1 mg/kg Cr;
calculated on the dry total weight of the bleached tobacco raw material.

In one embodiment the bleached tobacco raw material comprises a combined amount of aflatoxins B1, B2, G1, and G2 that does not exceed 0.01 mg/kg, calculated on the dry total weight of the bleached tobacco raw material.

In one embodiment the bleached tobacco raw material has an ISO brightness that is not less than about 60, specifically not less than about 70, and more specifically not less than about 80, as measured according to ISO 2470:1999.

As will be realized by the skilled person the present invention thus provides a tobacco raw material that is substantially free from undesired substances.

One advantage of the inventive bleached tobacco raw material is that it provides for tobacco products that does not discolour the user's fingers, and - in the case of oral smokeless tobacco products - does not discolour the user's teeth.

Another aspect of the invention relates to a smoking tobacco composition comprising the inventive bleached tobacco raw material.

In one embodiment the smoking tobacco composition comprises nicotine in addition to any nicotine naturally present in the tobacco. The added nicotine may be natural nicotine, i.e. nicotine extracted from tobacco plants, or synthetic nicotine, or a combination thereof.

In one embodiment the tobacco raw material content of the smoking tobacco composition comprises up to about 99 wt-%, specifically up to about 50 wt-%, and more specifically up to about 25 wt-% of unbleached tobacco raw material, calculated on the dry total weight of the tobacco raw material content of the composition.

Another aspect of the invention relates to a smokeless tobacco composition comprising the inventive bleached tobacco raw material.

In one embodiment the smokeless tobacco composition comprises nicotine in addition to any nicotine naturally present in the tobacco. The added nicotine may be natural nicotine, i.e. nicotine extracted from tobacco plants, or synthetic nicotine, or a combination thereof.

In one embodiment the tobacco raw material content of the smokeless tobacco composition comprises up to about 99 wt-%, specifically up to about 50 wt-%, and more specifically up to about 25 wt-% of unbleached tobacco raw material, calculated on the dry total weight of the tobacco raw material content of the composition.

Another aspect of the invention relates to nasal snuff, i.e. snuff products suited for nasal administration, which comprises the inventive smokeless tobacco composition.

Another aspect of the invention relates to an oral smokeless tobacco product comprising the inventive smokeless tobacco composition. The oral smokeless tobacco product may be, but is not limited to, moist snuff such as snus, chewing tobacco, oral dry snuff, or hard snuff.

A particular advantage of this aspect of the invention is that it the low content of fermentable carbohydrates provides for a greatly reduced risk for dental caries.

The oral smokeless tobacco product may comprise additives chosen among - but not limited to - one or more substances belonging to any of the following categories, or combinations thereof: API (active pharmaceutical substances), food additives, natural or synthetic nutrients, flavoring substances, natural medicaments or naturally occurring substances that can have an effect on humans. Examples of such substances are green tea, white tea, caffeine, vitamin B12, vitamin C, vitamin E, bioperin, Q10, selenium, glutathione, liponic acid, folic acid, ginseng, pollen extract, antioxidants, minerals, paracetamol, acetylsalicylic acid, Russian root, and rose root.

In one embodiment the oral smokeless tobacco product contains an alginate composition of the kind, and in the way described in WO 2010/114445, the contents of which is hereby incorporated by reference. More specifically, the oral smokeless tobacco product according to this embodiment contains an alginate composition, distributed in the product and comprising at least water, alginate and an added substance intended to be released from the product when said product is used, said composition containing an alginate matrix that retains at least a major proportion of the added substance so long as the matrix is intact, and the alginate matrix being formed so as to disintegrate and/or dissolve in the chemical and physical environment that exists in a user's mouth; wherein said alginate contains an alginate salt of monovalent cations and is soluble in cold water.

In one embodiment the oral smokeless tobacco product is chewing tobacco comprising a gum base. The gum base may be chosen among - but is not limited to - commercial chewing gum bases, bubble gum bases, and natural gum base materials.

The oral smokeless tobacco product may be provided in baked or compressed form, from which portions may punched or pressed in portions, such as lozenges or tablets.

As an alternative, the oral smokeless tobacco product may be provided in loose form or in the form of portion-size sachets. As a further alternative it may have a film-like form.

The oral smokeless tobacco product may have a pH of about 7-12, specifically about 8-12, more specifically about 8-9.

The oral smokeless tobacco product may have a water content of about 5 - 55%, specifically about 40 - 55%, more specifically about 45 - 52%.

Another aspect of the invention relates to the inventive bleached tobacco raw material for use in the treatment of nicotine addiction, such as for instance in a pharmaceutical for smoking cessation.

It is also disclosed a process for production of the inventive bleached tobacco raw material comprising the steps of:
(a) treating unbleached tobacco raw material at an acidic pH and at a temperature range of from about 70°C to about 180°C with an aqueous solution of comprising sulfite ion;
(b) defibrating the tobacco raw material from step (a);
   and
(c) treating the defibrated tobacco raw material from step (c) with an effective amount of a bleaching agent at a temperature range of from about 60°C to about 90°C.

The bleaching agent may be - but is not limited to - chlorine dioxide, sodium hypochlorite, hydrogen peroxide, ozone, enzymes, oxygen, or a combination thereof.

The bleaching agent may also be constituted by, or at least involve the use of ultrasound.

It is also disclosed a bleached tobacco raw material obtained or obtainable by said process, specifically such bleached tobacco raw material comprising less than about 4 weight-% fermentable carbohydrates, calculated on the dry total weight of the bleached tobacco raw material.

In one embodiment the bleached tobacco raw material obtained or obtainable by said process comprises less than about 2 weight-%, specifically less than about 1 weight-%, of fermentable carbohydrates, calculated on the dry total weight of the bleached tobacco raw material.

In one embodiment the bleached tobacco raw material obtained or obtainable by said process comprises less than about 7.0 mg/kg, specifically less than about 3.5 mg/kg, more specifically less than about 1.0 mg/kg of nitrite, calculated on the dry total weight of the bleached tobacco raw material.

In one embodiment the bleached tobacco raw material obtained or obtainable by said process comprises a combined amount of NNN (N-nitrosonornicotine) and NNK ((4-methylnitrosamino)-1-(3-pyridyl)-1-butanone) that is less than about 2.0 mg/kg, specifically less than about 1.0 mg/kg, more specifically less than about 0.2 mg/kg, calculated on the dry total weight of the bleached tobacco raw material.

In one embodiment the bleached tobacco raw material obtained or obtainable by said process comprises less than about 10.0 µg/kg, specifically less than about 5.0 µg/kg, more specifically less than about 0.6 µg/kg of NDMA (N-Nitrosodimethylamine), calculated on the dry total weight of the bleached tobacco raw material.

In one embodiment the bleached tobacco raw material obtained or obtainable by said process comprises less than about 5.0 µg/kg, specifically less than about 2.5 µg/kg, more specifically less than about 0.6 µg/kg of B(a)P (Benzo(a)pyrene), calculated on the dry total weight of the bleached tobacco raw material.

In one embodiment the bleached tobacco raw material obtained or obtainable by said process comprises less than about 1.0 mg/kg, specifically less than about 0.5 mg/kg, more specifically less than about 0.1 mg/kg of Cd, calculated on the dry total weight of the bleached tobacco raw material.

In one embodiment the bleached tobacco raw material obtained or obtainable by said process comprises less than about 2.0 mg/kg, specifically less than about 1.0 mg/kg, more specifically less than about 0.1 mg/kg of Pb, calculated on the dry total weight of the bleached tobacco raw material.

In one embodiment the bleached tobacco raw material obtained or obtainable by said process comprises less than about 0.5 mg/kg, specifically less than about 0.25 mg/kg, more specifically less than about 0.1 mg/kg of As, calculated on the dry total weight of the bleached tobacco raw material.

In one embodiment the bleached tobacco raw material obtained or obtainable by said process comprises less than about 3.0 mg/kg, specifically less than about 1.5 mg/kg, more specifically less than about 0.1 mg/kg of Ni, calculated on the dry total weight of the bleached tobacco raw material.

In one embodiment the bleached tobacco raw material obtained or obtainable by said process comprises less than about 4.5 mg/kg, specifically less than about 2.25 mg/kg, more specifically less than about 0.1 mg/kg of Cr, calculated on the dry total weight of the bleached tobacco raw material.

In one embodiment the bleached tobacco raw material obtained or obtainable by said process comprises one or more but not all of:
less than about 7.0 mg/kg nitrite;
less than about 2.0 mg/kg combined amount of NNN (N-nitrosonornicotine) and NNK ((4-methylnitrosamino)-1-(3-pyridyl)-1-butanone);
less than about 10.0 µg/kg NDMA (N-Nitrosodimethylamine);
less than about 5.0 µg/kg B(a)P (Benzo(a)pyrene);
less than about 1.0 mg/kg Cd;
less than about 2.0 mg/kg Pb;
less than about 0.5 mg/kg As;
less than about 3.0 mg/kg Ni;
less than about 4.5 mg/kg Cr;
calculated on the dry total weight of the bleached tobacco raw material.

In one embodiment the bleached tobacco raw material obtained or obtainable said process comprises one or more but not all of:
less than about 3.5 mg/kg nitrite;
less than about 1.0 mg/kg combined amount of NNN (N-nitrosonornicotine) and NNK ((4-methylnitrosamino)-1-(3-pyridyl)-1-butanone);
less than about 5.0 µg/kg NDMA (N-Nitrosodimethylamine);
less than about 2.5 µg/kg B(a)P (Benzo(a)pyrene);
less than about 0.5 mg/kg Cd;
less than about 1.0 mg/kg Pb;
less than about 0.25 mg/kg As;
less than about 1.5 mg/kg Ni;
less than about 2.25 mg/kg Cr;
calculated on the dry total weight of the bleached tobacco raw material.

In one embodiment the bleached tobacco raw material obtained or obtainable by said process comprises one or more but not all of:
less than about 1.0 mg/kg nitrite;
less than about 0.2 mg/kg combined amount of NNN (N-nitrosonornicotine) and NNK ((4-methylnitrosamino)-1-(3-pyridyl)-1-butanone);
less than about 0.6 µg/kg NDMA (N-nitrosodimethylamine);
less than about 0.6 µg/kg B(a)P (benzo(a)pyrene);
less than about 0.1 mg/kg Cd;
less than about 0.1 mg/kg Pb;
less than about 0.1 mg/kg As;
less than about 0.1 mg/kg Ni;
less than about 0.1 mg/kg Cr;
calculated on the dry total weight of the bleached tobacco raw material.

In one embodiment the bleached tobacco raw material obtained or obtainable by said process comprises a combined amount of aflatoxins B1, B2, G1, and G2 that does not exceed 0.01 mg/kg, calculated on the dry total weight of the bleached tobacco raw material.

In one embodiment the bleached tobacco raw material obtained or obtainable by said process has an ISO brightness that is not less than about 60, specifically not less than about 70, and more specifically not less than about 80, as measured according to ISO 2470:1999.

Another aspect of the invention relates to a smoking tobacco composition comprising the bleached tobacco raw material obtained or obtainable by said process.

In one embodiment this smoking tobacco composition comprises nicotine in addition to any nicotine naturally present in the tobacco. The added nicotine may be natural nicotine, i.e. nicotine extracted from tobacco plants, or synthetic nicotine, or a combination thereof.

In one embodiment the content of tobacco raw material obtained or obtainable by said process in this smoking tobacco composition comprises up to about 99 wt-%, specifically up to about 50 wt-%, and more specifically up to about 25 wt-% of unbleached tobacco raw material, calculated on the dry total weight of the tobacco raw material content of the composition.

Another aspect of the invention relates to a smokeless tobacco composition comprising the bleached tobacco raw material obtained or obtainable by said process.

In one embodiment this smokeless tobacco composition comprises nicotine in addition to any nicotine naturally present in the tobacco. The added nicotine may be natural nicotine, i.e. nicotine extracted from tobacco plants, or synthetic nicotine, or a combination thereof.

In one embodiment the tobacco raw material content of this smokeless tobacco composition comprises up to about 99 wt-%, specifically up to about 50 wt-%, and more specifically up to about 25 wt-% of unbleached tobacco raw material, calculated on the dry total weight of the tobacco raw material content of the composition.

Another aspect of the invention relates to nasal snuff, i.e. snuff products suited for nasal administration, which comprises this smokeless tobacco composition.

Another aspect of the invention relates to an oral smokeless tobacco product comprising this smokeless tobacco composition. The oral smokeless tobacco product may be, but is not limited to, moist snuff such as snus, chewing tobacco, oral dry snuff, or hard snuff.

This oral smokeless tobacco product may comprise additives chosen among - but not limited to - one or more substances belonging to any of the following categories, or combinations thereof: API (active pharmaceutical substances), food additives, natural or synthetic nutrients, flavoring substances, natural medicaments or naturally occurring substances that can have an effect on humans. Examples of such substances are green tea, white tea, caffeine, vitamin B12, vitamin C, vitamin E, bioperin, Q10, selenium, glutathione, liponic acid, folic acid, ginseng, pollen extract, antioxidants, minerals, paracetamol, acetylsalicylic acid, Russian root, and rose root.

In one embodiment this oral smokeless tobacco product contains an alginate composition of the kind, and in the way described in WO 2010/114445, the contents of which is hereby incorporated by reference. More specifically, the oral smokeless tobacco product according to this embodiment contains an alginate composition, distributed in the product and comprising at least water, alginate and an added substance intended to be released from the product when said product is used, said composition containing an alginate matrix that retains at least a major proportion of the added substance so long as the matrix is intact, and the alginate matrix being formed so as to disintegrate and/or dissolve in the chemical and physical environment that exists in a user's mouth; wherein said alginate contains an alginate salt of monovalent cations and is soluble in cold water.

In one embodiment this oral smokeless tobacco product is chewing tobacco comprising a gum base. The gum base may be chosen among - but is not limited to - commercial chewing gum bases, bubble gum bases, and natural gum base materials.

This oral smokeless tobacco product may be provided in baked or compressed form, from which portions may punched or pressed in portions, such as lozenges or tablets.

As an alternative, this oral smokeless tobacco product may be provided in loose form or in the form of portion-size sachets. As a further alternative it may have a film-like form.

This oral smokeless tobacco product may have a pH of about 7-12, specifically about 8-12, more specifically about 8-9.

This oral smokeless tobacco product may have a water content of about 5 - 55%, specifically about 40 - 55%, more specifically about 45 - 52%.

Another aspect of the invention relates to the bleached tobacco raw material for use in the treatment of nicotine addiction, such as for instance in a pharmaceutical for smoking cessation.

The invention will now be illustrated in closer detail in the following non-limiting examples.

### Example

### Digestion

### Step 1.

Unbleached tobacco raw material was cooked with water in a digesting vessel at 120°C for 30 minutes, after which the liquid was drawn off, and the material was flushed with water.

### Step 2.

Cooking liquor, with pH adjusted to 4.5 by SO₂, was added to the digesting vessel. The cooking liquor contained mainly sodium bisulfite, but also partly sodium sulfite. The cooking was performed at 165°C for 40 minutes, after which the digesting vessel was degassed. Water was added and circulated at 80°C. The digesting vessel was emptied and the material was flushed.

### Screening

The digested material was taken to a defibrator, in which the fibres were freed by beating.

The obtained fiber suspension was led to a screen with 0.25 mm slots to screen away undigested fibres. The remaining fibres were brought to a centrifuge in which the material, pulp, was dewatered to dry content of about 30%.

### Bleaching

The bleaching was carried out in a plastic vessel heated by a water bath.

### Step 1.

The pulp was treated at 70°C for 30 minutes with ClO₂ in an amount of 38 kg/ton of pulp, at a pulp concentration of 10%.

### Step 2.

The pulp was treated at 80°C for 120 minutes with ClO₂ in an amount of 38 kg/ton of pulp, at a pulp concentration of 10%.

### Step 3.

The pulp was treated at 80°C for 120 minutes with ClO₂ in an amount of 19 kg/ton of pulp, at a pulp concentration of 10%.

The pulp was washed after each step according to the following:
Bleaching liquor was drawn off in a centrifuge; the pulp was diluted with water and slurried by means of a stirrer; and the pulp was then washed in the centrifuge with 1 liter of water per 10 grams of pulp.

### Analysis

The resulting pulp was analyzed at Eurofins Food & Agro Testing Norway AS (Skansen), in Trondheim, Norway, with respect to the content of starches and simple sugars, which proved to be <1.0%.

The pulp was also analyzed at Eurofins Food & Agro Testing Sweden AB, Lidköping, Sweden, with resepct to the content of sucrose, which proved to be <0.04 g/100 g, i.e. <0.04%.

Thus, the total content of fermentable carbohydrates <1.04%.

The pulp was also analyzed at Eurofins Food & Agro Testing Sweden AB with respect to the content of nitrite, which proved to be <1.0 mg/kg.

The pulp was also analyzed at Eurofins Food & Agro Testing Sweden AB with respect to the combined amount of NNN (N-nitrosonornicotine) and NNK ((4-methylnitrosamino)-1-(3-pyridyl)-1-butanone), which proved to be <0.020 mg/kg.

The pulp was also analyzed at Eurofins Food & Agro Testing Sweden AB with respect to the content of NDMA (N-Nitrosodimethylamine), which proved to be <0.20 µg/kg.

The pulp was also analyzed at Eurofins Food & Agro Testing Sweden AB with respect to the content of B(a)P (Benzo(a)pyrene) which proved to be <1.0 µg/kg.

The pulp was also analyzed at Eurofins Environment Sweden, Lidköping, Sweden, with respect to the content of Cd, which proved to be 0.063 mg/kg ± 20% (analysis method/reference NMKL No 161 1998 mod).

The pulp was also analyzed at Eurofins Environment Sweden, Lidköping, Sweden, with respect to the content of Pb, which proved to be 0.63 mg/kg ± 20% (analysis method/reference NMKL No 161 1998 mod).

The pulp was also analyzed at Eurofins Environment Sweden, Lidköping, Sweden, with respect to the content of As, which proved to be <0.050 mg/kg ± 35% (analysis method/reference NMKL No 161 1998 mod).

The pulp was also analyzed at Eurofins Environment Sweden, Lidköping, Sweden, with respect to the content of Ni, which proved to be 0.63 mg/kg ± 25% (analysis method/reference NMKL No 161 1998 mod).

The pulp was also analyzed at Eurofins Environment Sweden, Lidköping, Sweden, with respect to the content of Cr, which proved to be 1.5 mg/kg ± 20% (analysis method/reference NMKL No 161 1998 mod).

Although the invention has been described with regard to certain embodiments it should be understood that various changes and modifications as would be obvious to one having the ordinary skill in this art may be made without departing from the scope of the invention as set forth in the claims appended hereto.

## Claims

1. A bleached tobacco raw material comprising less than 4 weight-% fermentable carbohydrates, calculated on the dry total weight of the bleached tobacco raw material, wherein the bleached tobacco raw material comprises less than 5.0 µg/kg of B(a)P (Benzo(a)pyrene); and one or more, but not all of:
less than 7.0 mg/kg of nitrite;
less than 2.0 mg/kg of a combined amount of NNN (N-nitrosonornicotine) and NNK ((4- methylnitrosamino)-1-(3-pyridyl)-1-butanone);
less than 10.0 µg/kg of NDMA (N-Nitrosodimethylamine);
less than 1.0 mg/kg of Cd;
less than 2.0 mg/kg of Pb;
less than 0.5 mg/kg of As;
less than 3.0 mg/kg of Ni; and
less than 4.5 mg/kg of Cr;
calculated on the dry total weight of the bleached tobacco raw material.

2. The bleached tobacco raw material according to claim 1, comprising less than 2 weight-% of fermentable carbohydrates, such as less than 1 weight-% of fermentable carbohydrates, calculated on the dry total weight of the bleached tobacco raw material.

3. The bleached tobacco raw material according to claim 1 or 2, comprising less than 2.5 µg/kg of B(a)P (Benzo(a)pyrene), calculated on the dry total weight of the bleached tobacco raw material.

4. The bleached tobacco raw material according to any one of the preceding claims, comprising less than 2.5 µg/kg B(a)P (benzo(a)pyrene); and one or more, but not all of:
less than 3.5 mg/kg nitrite;
less than 1.0 mg/kg combined amount of NNN (N-nitrosonornicotine) and NNK ((4-methylnitrosamino)-1-(3-pyridyl)-1-butanone);
less than 5.0 µg/kg NDMA (N-nitrosodimethylamine);
less than 0.5 mg/kg Cd;
less than 1.0 mg/kg Pb;
less than 0.25 mg/kg As;
less than 1.5 mg/kg Ni; and
less than 2.25 mg/kg Cr;
calculated on the dry total weight of the bleached tobacco raw material.

5. The bleached tobacco raw material according to any one of the preceding claims, comprising less than 0.6 µg/kg B(a)P (benzo(a)pyrene); and one or more, but not all of:
less than 1.0 mg/kg nitrite;
less than 0.2 mg/kg combined amount of NNN (N-nitrosonornicotine) and NNK ((4-methylnitrosamino)-1-(3-pyridyl)-1-butanone);
less than 0.6 µg/kg NDMA (N-nitrosodimethylamine);
less than 0.1 mg/kg Cd;
less than 0.1 mg/kg Pb;
less than 0.1 mg/kg As;
less than 0.1 mg/kg Ni; and
less than 0.1 mg/kg Cr;
calculated on the dry total weight of the bleached tobacco raw material.

6. The bleached tobacco raw material according to any one of the preceding claims, comprising a combined amount of aflatoxins B1, B2, G1, and G2 that does not exceed 0.01 mg/kg, calculated on the dry total weight of the bleached tobacco raw material.

7. The bleached tobacco raw material according to any one of the preceding claims, where the bleached tobacco raw material has an ISO brightness that is not less than 60, such as not less than 70, such as not less than 80.

8. A smoking tobacco composition comprising the bleached tobacco raw material according to any one of the preceding claims.

9. The smoking tobacco composition according to claim 8, where the tobacco raw material content of the composition comprises up to 50 wt-% of unbleached tobacco raw material, calculated on the dry total weight of the tobacco raw material content of the composition.

10. A smokeless tobacco composition comprising the bleached tobacco raw material according to any one of claims 1 to 7.

11. The smokeless tobacco composition according to claim 10, wherein the tobacco raw material content of the composition comprises up to 50 wt-% unbleached tobacco raw material, calculated on the dry total weight of the tobacco raw material content of the composition.

12. Nasal snuff comprising the smokeless tobacco composition according to any one of claims 10 to 11.

13. An oral smokeless tobacco product comprising the smokeless tobacco composition according to any one of claims 10 to 11, where the oral smokeless tobacco product is moist snuff such as snus, or chewing tobacco, oral dry snuff or hard snuff.

14. The oral smokeless tobacco product according to claim 13, wherein the product contains an alginate composition, distributed in the product and comprising at least water, alginate and an added substance intended to be released from the product when said product is used, said composition containing an alginate matrix that retains at least a major proportion of the added substance so long as the matrix is intact, and the alginate matrix being formed so as to disintegrate and/or dissolve in the chemical and physical environment that exists in a user's mouth; wherein said alginate contains an alginate salt of monovalent cations and is soluble in cold water.

15. Bleached tobacco raw material according to any one of claims 1 to 7 for use in the treatment of nicotine addiction.

## Patentansprüche

1. Gebleichtes Tabakrohmaterial, das weniger als 4 Gew.-% fermentierbare Kohlenhydrate umfasst, berechnet auf dem Trockengesamtgewicht des gebleichten Tabakrohmaterials, wobei das gebleichte Tabakrohmaterial weniger als 5,0 µg/kg B(a)P (Benzo(a)pyren) umfasst, und eines oder mehrere, aber nicht alles von:
weniger als 7,0 mg/kg Nitrit;
weniger als 2,0 mg/kg einer kombinierten Menge an NNN (N-Nitrosonornicotin) und NNK ((4-Methylnitrosamino)-1-(3-pyridyl)-1-butanon);
weniger als 10,0 µg/kg NDMA (N-Nitrosodimethylamin);
weniger als 1,0 mg/kg Cd;
weniger als 2,0 mg/kg Pb;
weniger als 0,5 mg/kg As;
weniger als 3,0 mg/kg Ni; und
weniger als 4,5 mg/kg Cr;
berechnet auf dem Trockengesamtgewicht des gebleichten Tabakrohmaterials.

2. Gebleichtes Tabakrohmaterial nach Anspruch 1, umfassend weniger als 2 Gew.-% fermentierbare Kohlenhydrate, wie weniger als 1 Gew.-% fermentierbare Kohlenhydrate, berechnet auf dem Trockengesamtgewicht des gebleichten Tabakrohmaterials.

3. Gebleichtes Tabakrohmaterial nach Anspruch 1 oder 2, umfassend weniger als 2,5 µg/kg B(a)P (Benzo(a)pyren), berechnet auf dem Trockengesamtgewicht des gebleichten Tabakrohmaterials.

4. Gebleichtes Tabakrohmaterial nach einem der vorhergehenden Ansprüche, umfassend weniger als 2,5 µg/kg B(a)P (Benzo(a)pyren); und eines oder mehrere, aber nicht alles von:
weniger als 3,5 mg/kg Nitrit;
weniger als 1,0 mg/kg kombinierte Menge an NNN (N-Nitrosonornicotin) und NNK ((4-Methylnitrosamino)-1-(3-pyridyl)-1-butanon);
weniger als 5,0 µg/kg NDMA (N-Nitrosodimethylamin);
weniger als 0,5 mg/kg Cd;
weniger als 1,0 mg/kg Pb;
weniger als 0,25 mg/kg As;
weniger als 1,5 mg/kg Ni; und
weniger als 2,25 mg/kg Cr;
berechnet auf dem Trockengesamtgewicht des gebleichten Tabakrohmaterials.

5. Gebleichtes Tabakrohmaterial nach einem der vorhergehenden Ansprüche, umfassend weniger als 0,6 µg/kg B(a)P (Benzo(a)pyren); und eines oder mehrere, aber nicht alles von:
weniger als 1,0 mg/kg Nitrit;
weniger als 0,2 mg/kg kombinierte Menge an NNN (N-Nitrosonornicotin) und NNK ((4-Methylnitrosamino)-1-(3 -pyridyl)-1-butanon);
weniger als 0,6 µg/kg NDMA (N-Nitrosodimethylamin);
weniger als 0,1 mg/kg Cd;
weniger als 0,1 mg/kg Pb;
weniger als 0,1 mg/kg As;
weniger als 0,1 mg/kg Ni; und
weniger als 0,1 mg/kg Cr;
berechnet auf dem Trockengesamtgewicht des gebleichten Tabakrohmaterials.

6. Gebleichtes Tabakrohmaterial nach einem der vorhergehenden Ansprüche, umfassend eine kombinierte Menge an Aflatoxinen B1, B2, G1 und G2, die 0,01 mg/kg, berechnet auf dem Trockengesamtgewicht des gebleichten Tabakrohmaterials, nicht überschreitet.

7. Gebleichtes Tabakrohmaterial nach einem der vorhergehenden Ansprüche, wobei das gebleichte Tabakrohmaterial eine ISO-Helligkeit aufweist, die nicht weniger als 60, wie nicht weniger als 70, wie nicht weniger als 80 ist.

8. Rauchtabakzusammensetzung, umfassend das gebleichte Tabakrohmaterial nach einem der vorhergehenden Ansprüche.

9. Rauchtabakzusammensetzung nach Anspruch 8, wobei der Tabakrohmaterialgehalt der Zusammensetzung bis zu 50 Gew.-% ungebleichtes Tabakrohmaterial umfasst, berechnet auf dem Trockengesamtgewicht des Tabakrohmaterialgehalts der Zusammensetzung.

10. Rauchfreie Tabakzusammensetzung, umfassend das gebleichte Tabakrohmaterial nach einem der Ansprüche 1 bis 7.

11. Rauchfreie Tabakzusammensetzung nach Anspruch 10, wobei der Tabakrohmaterialgehalt der Zusammensetzung bis zu 50 Gew.-% ungebleichtes Tabakrohmaterial umfasst, berechnet auf dem Trockengesamtgewicht des Tabakrohmaterialgehalts der Zusammensetzung.

12. Nasaler Schnupftabak, umfassend die rauchfreie Tabakzusammensetzung nach einem der Ansprüche 10 bis 11.

13. Orales rauchfreies Tabakprodukt, umfassend die rauchfreie Tabakzusammensetzung nach einem der Ansprüche 10 bis 11, wobei das orale rauchfreie Tabakprodukt feuchter Schnupftabak wie Snus oder Kautabak, oraler trockener Schnupftabak oder harter Schnupftabak ist.

14. Orales rauchfreies Tabakprodukt nach Anspruch 13, wobei das Produkt eine Alginatzusammensetzung enthält, die in dem Produkt verteilt ist und zumindest Wasser, Alginat und eine hinzugefügte Substanz umfasst, die aus dem Produkt freigesetzt werden soll, wenn das Produkt verwendet wird, wobei die Zusammensetzung eine Alginatmatrix enthält, die zumindest einen Großteil der hinzugefügten Substanz zurückhält, solange die Matrix intakt ist, und die Alginatmatrix gebildet ist, sodass sie in der chemischen und physikalischen Umgebung, die in dem Mund eines Benutzers herrscht, zerfällt und/oder sich auflöst; wobei das Alginat ein Alginatsalz von einwertigen Kationen enthält und löslich in kaltem Wasser ist.

15. Gebleichtes Tabakrohmaterial nach einem der Ansprüche 1 bis 7 zur Verwendung bei der Behandlung von Nikotinsucht.

## Revendications

1. Matériau brut à base de tabac blanchi comprenant moins de 4 % en poids de glucides fermentables, calculés par rapport au poids total sec du matériau brut à base de tabac blanchi, ledit matériau brut à base de tabac blanchi comprenant moins de 5,0 µg/kg de B(a)P (benzo(a)pyrène) ; et un ou plusieurs composés, mais pas tous parmi :
moins de 7,0 mg/kg de nitrite ;
moins de 2,0 mg/kg d'une quantité combinée de NNN (N-nitrosonornicotine) et de NNK ((4-méthylnitrosamino)-1-(3 -pyridyl)-1-butanone) ;
moins de 10,0 µg/kg de NDMA (N-nitrosodiméthylamine) ;
moins de 1,0 mg/kg de Cd ;
moins de 2,0 mg/kg de Pb ;
moins de 0,5 mg/kg d'As ;
moins de 3,0 mg/kg de Ni ; et
moins de 4,5 mg/kg de Cr ;
calculés par rapport au poids total sec du matériau brut à base de tabac blanchi.

2. Matériau brut à base de tabac blanchi selon la revendication 1, comprenant moins de 2 % en poids de glucides fermentables, tels que moins de 1 % en poids de glucides fermentables, calculés par rapport au poids total sec du matériau brut à base de tabac blanchi.

3. Matériau brut à base de tabac blanchi selon la revendication 1 ou 2, comprenant moins de 2,5 µg/kg de B(a)P (benzo(a)pyrène), calculés par rapport au poids total sec du matériau brut à base de tabac blanchi.

4. Matériau brut à base de tabac blanchi selon l'une quelconque des revendications précédentes, comprenant moins de 2,5 µg/kg de B(a)P (benzo(a)pyrène) ; et un ou plusieurs composés, mais pas tous parmi :
moins de 3,5 mg/kg de nitrite ;
moins de 1,0 mg/kg d'une quantité combinée de NNN (N-nitrosonornicotine) et de NNK ((4-méthylnitrosamino)-1-(3-pyridyl)-1-butanone) ;
moins de 5,0 µg/kg de NDMA (N-nitrosodiméthylamine) ;
moins de 0,5 mg/kg de Cd ;
moins de 1,0 mg/kg de Pb ;
moins de 0,25 mg/kg d'As ;
moins de 1,5 mg/kg de Ni ; et
moins de 2,25 mg/kg de Cr ;
calculés par rapport au poids total sec du matériau brut à base de tabac blanchi.

5. Matériau brut à base de tabac blanchi selon l'une quelconque des revendications précédentes, comprenant moins de 0,6 µg/kg de B(a)P (benzo(a)pyrène) ; et un ou plusieurs composés, mais pas tous parmi :
moins de 1,0 mg/kg de nitrite ;
moins de 0,2 mg/kg d'une quantité combinée de NNN (N-nitrosonornicotine) et de NNK ((4-méthylnitrosamino)-1-(3 -pyridyl)-1-butanone) ;
moins de 0,6 µg/kg de NDMA (N-nitrosodiméthylamine) ;
moins de 0,1 mg/kg de Cd ;
moins de 0,1 mg/kg de Pb ;
moins de 0,1 mg/kg d'As ;
moins de 0,1 mg/kg de Ni ; et
moins de 0,1 mg/kg de Cr ;
calculés par rapport au poids total sec du matériau brut à base de tabac blanchi.

6. Matériau brut à base de tabac blanchi selon l'une quelconque des revendications précédentes, comprenant une quantité combinée d'aflatoxines B1, B2, G1 et G2 qui ne dépasse pas 0,01 mg/kg, calculé par rapport au poids total sec du matériau brut à base de tabac blanchi.

7. Matériau brut à base de tabac blanchi selon l'une quelconque des revendications précédentes, ledit matériau brut à base de tabac blanchi comportant une blancheur ISO qui est non inférieure à 60, telle que non inférieure à 70, telle que non inférieure à 80.

8. Composition de tabac à fumer comprenant le matériau brut à base de tabac blanchi selon l'une quelconque des revendications précédentes.

9. Composition de tabac à fumer selon la revendication 8, la teneur en matériau brut à base de tabac de la composition comprenant jusqu'à 50 % en poids de matériau brut à base de tabac non blanchi, calculés par rapport au poids total sec de la teneur en matériau brut à base de tabac de la composition.

10. Composition de tabac sans fumée comprenant le matériau brut à base de tabac blanchi selon l'une quelconque des revendications 1 à 7.

11. Composition de tabac sans fumée selon la revendication 10, ladite teneur en matériau brut à base de tabac de la composition comprenant jusqu'à 50 % en poids de matériau brut à base de tabac non blanchi, calculés par rapport au poids total sec de la teneur en matériau brut à base de tabac de la composition.

12. Tabac à priser nasal comprenant la composition de tabac sans fumée selon l'une quelconque des revendications 10 à 11.

13. Produit de tabac sans fumée oral comprenant la composition de tabac sans fumée selon l'une quelconque des revendications 10 à 11, ledit produit de tabac sans fumée oral étant du tabac à priser humide tel que du tabac à chiquer ou du tabac à mâcher, du tabac à priser sec oral ou du tabac dur à priser.

14. Produit de tabac sans fumée oral selon la revendication 13, ledit produit contenant une composition d'alginate, répartie dans le produit et comprenant au moins de l'eau, de l'alginate et une substance ajoutée destinée à être libérée du produit lorsque ledit produit est utilisé, ladite composition contenant une matrice d'alginate qui retient au moins une majeure partie de la substance ajoutée tant que la matrice est intacte, et ladite matrice d'alginate étant formée de façon à se désintégrer et/ou se dissoudre dans l'environnement chimique et physique qui existe dans la bouche d'un utilisateur ; ledit alginate contenant un sel d'alginate de cations monovalents et étant soluble dans l'eau froide.

15. Matériau brute à base de tabac blanchi selon l'une quelconque des revendications 1 à 7 destiné à être utilisé dans le traitement de l'addiction à la nicotine.
